# EUROPÄISCHE PATENTSCHRIFT

(11) **EP 3 331 587 B1**
(45) Veröffentlichungstag und Bekanntmachung des Hinweises auf die Patenterteilung: **02.09.2020**
(21) Anmeldenummer: 16742326.8
(22) Anmeldetag: 26.07.2016
(51) Int. Cl.: A61M 1/16, A61M 1/34

(54) **PORTABLE ULTRAFILTRATIONSEINHEIT UND VORRICHTUNG ZUR VERSORGUNG DER ULTRAFILTRATIONSEINHEIT MIT DIALYSIERFLÜSSIGKEIT**
PORTABLE ULTRAFILTRATION UNIT AND DEVICE FOR SUPPLYING THE ULTRAFILTRATION UNIT WITH DIALYSIS FLUID
UNITÉ D'ULTRAFILTRATION PORTATIVE ET DISPOSITIF D'ALIMENTATION DE L'UNITÉ D'ULTRAFILTRATION EN LIQUIDE DE DIALYSE

(30) Priorität: 06.08.2015 DE 102015010316
(43) Veröffentlichungstag der Anmeldung: 13.06.2018
(73) Patentinhaber: Fresenius Medical Care Deutschland GmbH, 61352 Bad Homburg v.d.H. (DE)
(72) Erfinder: PETERS, Arne, 61352 Bad Homburg (DE)
(74) Vertreter: Oppermann, Frank
(86) Internationale Anmeldenummer: PCT/EP2016/067834
(87) Internationale Veröffentlichungsnummer: WO 2017/021236

(56) Entgegenhaltungen:
- WO-A2-2009/083011
- US-A- 4 269 708
- US-A1- 2004 254 514
- US-A1- 2009 120 864
- US-A1- 2013 213 890
- US-A1- 2013 292 312

## Beschreibung

Die Erfindung betrifft ein medizinisches Behandlungssystem mit einer portablen Ultrafiltrationseinheit und einer stationären Vorrichtung zur Versorgung der Ultrafiltrationseinheit mit Dialysierflüssigkeit.

Zur Entfernung von harnpflichtigen Substanzen und zum Flüssigkeitsentzug werden beim chronischen Nierenversagen verschiedene Verfahren zur apparativen Blutbehandlung eingesetzt. Bei der Hämodialyse (HD) überwiegt der diffusive Stofftransport über die semipermeable Membran eines Filters, während bei der Hämofiltration (HF) ein konvektiver Stofftransport über die Filtermembran stattfindet. Eine Kombination aus beiden Verfahren ist die Hämodiafiltration (HDF).

Die bekannten Vorrichtungen zur Durchführung der genannten Blutbehandlungsverfahren weisen einen extrakorporalen Blutkreislauf und ein Dialysierflüssigkeitssystem auf. Der extrakorporale Blutkreislauf umfasst eine arterielle Blutleitung, die zu einer Blutkammer eines von einer semipermeablen Membran in eine erste und eine zweite Kammer unterteilten Filters (Dialysator) führt, und eine venöse Blutleitung, die von der ersten Kammer abgeht. Das Dialysierflüssigskeitssystem umfasst eine zu der zweiten Kammer des Filters führende Dialysierflüssigkeitszuführleitung und eine von der zweiten Kammer des Filters abgehende Dialysierflüssigkeitsabführleitung.

Wegen der großen Austauschmengen besteht die Notwendigkeit der exakten Bilanzierung der Menge der dem Patienten zugeführten Flüssigkeit und der entzogenen Flüssigkeit über die gesamte Behandlungszeit. Daher verfügen die bekannten Vorrichtungen zur extrakorporalen Blutbehandlung über eine Bilanzierungseinheit.

Die extrakorporalen Blutbehandlungen werden beispielsweise in zeitlichen Abständen von 2 bis 3 Tagen durchgeführt. Ziel einer optimalen Nierenersatztherapie ist neben der Reinigung des Blutes auch die Einstellung des Blutwasservolumens. Während der Blutbehandlung wird dem extrakorporalen Blutkreislauf daher über die Filtermembran eine gezielte Menge an Ultrafiltrat entzogen. Beispielsweise werden dem Patienten ca. 3 Liter Flüssigkeit entzogen. Nach dem Entzug der Flüssigkeit steigt das Blutwasservolumen zwischen den Behandlungen wieder an. Bei Patienten mit einem stark überhöhten Blutwasservolumen kann es vorkommen, dass die Blutbehandlung nicht innerhalb der vorgesehenen Zeit abgeschlossen werden kann, da das angestrebte Ultrafiltrationsziel noch nicht erreicht ist.

Für eine optimale Nierenersatztherapie wären Blutbehandlungen in möglichst kurzen Zeitabständen vorteilhaft. Eine Verkürzung der Zeitabstände zwischen den Behandlungen stellt aber nicht nur eine große Belastung für den Patienten, sondern auch ein logistisches Problem dar.

Neben den stationären Blutbehandlungsvorrichtungen, die eine Durchführung von verschiedenen Blutbehandlungen erlauben, beispielsweise eine Hämodialyse (HD), Hämofiltration (HF) oder Hämodiafiltration (HDF), sind auch portable Vorrichtungen bekannt, mit denen sich aber nur eine Hämofiltration (HF) durchführen lassen. Die portablen Ultrafiltrationsvorrichtungen umfassen nur die für eine Ultrafiltration erforderlichen Komponenten einer stationären Blutbehandlungsvorrichtung. Daher kann die Ultrafiltration mit einem verhältnismäßig geringen technischen Aufwand erfolgen.

Die WO 2009/083011 A2 beschreibt eine Vorrichtung zur Durchführung einer Dialysebehandlung, die über eine Dialyseeinheit (Dialysator) verfügt. Während der Dialysebehandlung wird die Dialysatkammer des Dialysators von Dialysat durchströmt. Gleichzeitig findet eine Ultrafiltration statt. Darüber hinaus verfügt die Dialysevorrichtung über eine Einrichtung zur Aufbereitung des gebrauchten Dialysats, die eine Osmose-Einheit und eine Harnstoff-Entfernungseinheit aufweist. Die WO 2009/083011 A2 schlägt vor, die für die Dialysebehandlung erforderlichen Komponenten in einer am Körper zu tragenden Einheit und die für die Aufbereitung des Dialysats erforderlichen Komponenten in einer transportierbaren Einheit unterzubringen, wobei eine Verbindung zwischen der tragbaren und transportierbaren Einheit hergestellt werden soll.

Aus der US 2009/120864 A1 ist ein medizinisches Behandlungssystem mit einer am Körper zu tragenden Einheit und einer transportierbaren Einheit bekannt, die mittels lösbaren Schlauchkupplungen miteinander verbunden werden können. Die am Körper zu tragende Einheit umfasst einen Dialysator sowie ein Steuergerät und die transportierbare Einheit eine Einrichtung zur Aufbereitung von gebrauchtem Dialysat. Während der Blutbehandlung strömt aufbereitetes Dialysat in den Einlass und gebrauchtes Dialysat aus dem Auslass der Dialysatkammer des Dialysators. Die am Körper zu tragende Einheit ist dazu bestimmt, zusammen mit der transportierbaren Einheit betrieben zu werden. Die US 2009/120864 A1 sieht zwar auch einen Betrieb vor, bei dem beide Einheiten voneinander getrennt sind, so dass sich der Patient frei bewegen kann. In diesem Betrieb ist aber eine Versorgung des Dialysators mit Dialysat nicht gegeben, so dass die Dialysebehandlung unterbrochen ist und nur das Blut durch die Blutkammer des Dialysators zirkuliert.

Die US 2013/213890 A1 beschreibt eine portable Vorrichtung zur Ultrafiltration, die über einen extrakorporalen Blutkreislauf mit einem Filter (Dialysator) und einer Blutpumpe verfügt. Der Dialysator weist einen Anschluss auf, an den eine Filtratleitung angeschlossen ist, die zu einem Filtratbehälter führt. Zum Fördern von Filtrat ist in der Filtratleitung eine Filtratpumpe vorgesehen. Die portable Ultrafiltrationsvorrichtung kann zusammen mit einer Reinigungsvorrichtung betrieben werden, die einen Dialysierflüssigkeitskreislauf mit einer Filterkartusche zur Entfernung von Harnstoff aus der Dialysierflüssigkeit und einer Dialysierflüssigkeitspumpe aufweist. Die Reinigungsvorrichtung kann auch eine portable Einheit sein.

Der Erfindung liegt die Aufgabe zugrunde, den apparativen und zeitlichen Aufwand für die Durchführung von Blutbehandlungen weiter zu verringern.

Die Lösung dieser Aufgabe erfolgt erfindungsgemäß mit den Merkmalen der unabhängigen Patentansprüche. Die abhängigen Ansprüche betreffen vorteilhafte Ausführungsformen der Erfindung.

Die erfindungsgemäße Ultrafiltrationseinheit des medizinischen Behandlungssystems erlaubt als autarke Einheit die Durchführung einer Hämofiltrationsbehandlung (Ultrafiltration) mit einem verhältnismäßig geringen apparativen Aufwand, da die Ultrafiltrationseinheit nur über die für die Ultrafiltration erforderlichen Komponenten verfügt. Der Patient kann mit der Ultrafiltrationseinheit nicht nur in der Klinik, sondern auch zu Hause behandelt werden. Mit der Ultrafiltrationseinheit können zwischen den Blutbehandlungen mit einer stationären Dialysemaschine zusätzliche Ultrafiltrationen in kurzen Zeitabständen erfolgen. Die Ultrafiltrationseinheit kann der Patient als portable Einheit mitnehmen. Der einfache apparative Aufbau der Ultrafiltrationseinheit ermöglicht einen Batteriebetrieb.

Die erfindungsgemäße Ultrafiltrationseinheit erlaubt aber nicht nur den Entzug von Flüssigkeit in einem Zeitraum zwischen den Blutbehandlungen, sondern ist auch als eine Einheit zum Anschluss an eine stationäre Vorrichtung zur Versorgung der Ultrafiltrationseinheit mit Dialysierflüssigkeit derart ausgebildet, dass eine Flüssigkeitsverbindung für die Zufuhr von frischer und Abfuhr von verbrauchter Dialysierflüssigkeit herstellbar ist. Folglich können mit der Ultrafiltrationseinheit nicht nur eine Hämofiltrationsbehandlung (Ultrafiltration), sondern in Verbindung mit der stationären Vorrichtung zur Versorgung der Ultrafiltrationseinheit mit Dialysierflüssigkeit auch Blutbehandlungen wie mit einer konventionellen Blutbehandlungsvorrichtung durchgeführt werden, beispielsweise eine Hämodialyse (HD).

In Verbindung mit der stationären Vorrichtung zur Versorgung der Ultrafiltrationseinheit mit Dialysierflüssigkeit ergeben sich weitere Vorteile. Für die Durchführung einer Blutbehandlung ist es nur erforderlich, die Ultrafiltrationseinheit mit der Vorrichtung zur Versorgung der Ultrafiltrationseinheit mit Dialysierflüssigkeit zu verbinden. Dabei ist es nicht erforderlich, die Patientenanschlüsse der venösen und arteriellen Blutleitungen zu lösen bzw. an den Patienten anzuschließen. Da der Patient an der Ultrafiltrationseinheit angeschlossen bleibt, kann die Vorbereitungszeit für eine Blutbehandlung mit der portablen Ultrafiltrationseinheit stark verkürzt werden.

Die erfindungsgemäße Vorrichtung zur Versorgung der Ultrafiltrationseinheit mit Dialysierflüssigkeit verfügt über eine Einrichtung zur Bereitstellung von frischer Dialysierflüssigkeit, eine Einrichtung zum Abführen von verbrauchter Dialysierflüssigkeit und eine Bilanzierungseinheit zum Bilanzieren von frischer und verbrauchter Dialysierflüssigkeit, wobei die Vorrichtung zur Versorgung der Ultrafiltrationseinheit mit Dialysierflüssigkeit derart ausgebildet ist, dass eine Flüssigkeitsverbindung für die Zufuhr von frischer Dialysierflüssigkeit zu der Ultrafiltrationseinheit und Abfuhr von verbrauchter Dialysierflüssigkeit von der Ultrafiltrationseinheit herstellbar ist.

Das erfindungsgemäße medizinische Behandlungssystem können eine oder mehrere portable Ultrafiltrationseinheiten und eine oder mehrere Vorrichtungen zur Versorgung der Ultrafiltrationseinheit mit Dialysierflüssigkeit bilden.

Die erfindungsgemäße Ultrafiltrationseinheit kann an eine Vorrichtung angeschlossen werden, die nur die für Bereitstellung von frischer Dialysierflüssigkeit und Abfuhr von verbrauchter Dialysierflüssigkeit sowie Bilanzierung von frischer und verbrauchter Dialysierflüssigkeit erforderlichen Baugruppen enthält. Die Vorrichtung zur Versorgung der Ultrafiltrationseinheit mit Dialysierflüssigkeit kann aber auch als eine Vorrichtung zur extrakorporalen Blutbehandlung ausgebildet sein, die einen extrakorporalen Blutkreislauf und ein Dialysierflüssigkeitssystem aufweist. Die Ultrafiltrationseinheit macht dann aber nur von den Komponenten für die Bereitstellung, Abfuhr und Bilanzierung der Dialysierflüssigkeit Gebrauch. Der gesamte extrakorporale Blutkreislauf oder zumindest Teile des extrakorporalen Blutkreislaufs können ungenutzt bleiben. Diese Ausführungsform der Vorrichtung zur Versorgung der Ultrafiltrationseinheit mit Dialysierflüssigkeit hat den Vorteil, dass die Vorrichtung auch ohne die Ultrafiltrationseinheit betrieben werden kann.

Für das Grundprinzip der Erfindung ist nicht wesentlich, mit welchen Komponenten eine Flüssigkeitsverbindung hergestellt wird. Die Ultrafiltrationseinheit weist vorzugsweise einen ersten Dialysat-Anschluss für eine Zuführleitung für frisches Dialysat und einen zweiten Dialysat-Anschluss für eine Abführleitung für verbrauchtes Dialysat auf. Die Dialysat Anschlüsse können unterschiedlich ausgebildet sein. Die Flüssigkeitsverbindung sollte sich einfach und sicher herstellen lassen. Hierzu können die bekannten Konnektoren Verwendung finden, beispielsweise die bekannten Hansen-Kupplungen. Zur Herstellung einer Flüssigkeitsverbindung für die Zufuhr von frischer Dialysierflüssigkeit zu der Ultrafiltrationseinheit und Abfuhr von verbrauchter Dialysierflüssigkeit von der Ultrafiltrationseinheit ist vorzugsweise eine Andockstation vorgesehen, so dass sich die Ultrafiltrationseinheit einfach und sicher an die Versorgungsvorrichtung anschließen lässt.

Der Filter der Ultrafiltrationseinheit ist vorzugsweise ein austauschbarer Filter, der nur zu einer einmaligen Verwendung bestimmt ist (Disposable). Die bekannten Filter weisen eine erste Kammer und eine zweite Kammer auf, die von einer semipermeablen Membran getrennt sind. Die erste und zweite Kammer der bekannten Filter haben jeweils einen Einlass und einen Auslass. Zum Anschluss des Filters weist die Ultrafiltrationseinheit einen ersten Filter-Anschluss für den Einlass der ersten Kammer des Filters und einen zweiten Filter-Anschluss für den Auslass der ersten Kammer des Filters auf, und einen dritten Filter-Anschluss für den Einlass der zweiten Kammer des Filters und einen vierten Filter-Anschluss für den Auslass der zweiten Kammer des Filters auf.

Eine bevorzugte Ausführungsform der Ultrafiltrationseinheit sieht eine erste Dialysat-Verbindungsleitung, die den ersten Dialysat-Anschluss mit dem dritten Filter-Anschluss verbindet, und eine zweite Dialysat-Verbindungsleitung vor, die den zweiten Dialysat-Anschluss mit dem vierten Filter-Anschluss verbindet. Dadurch wird eine direkte Flüssigkeitsverbindung zwischen den betreffenden Komponenten hergestellt. Die Verbindungsleitungen können fest installierte oder austauschbare Schlauchleitungen sein. Für den Austausch der Leitungen können geeignete Konnektoren vorgesehen sein.

Eine weitere bevorzugte Ausführungsform sieht eine Fluidverbindung des zu dem Einlass der Ultrafiltrationspumpe führenden Ultrafiltrat-Leitungsabschnitts mit dem dritten Filter-Anschluss und/oder eine Fluidverbindung des zu dem Einlass der Ultrafiltrationspumpe führenden Ultrafiltrat-Leitungsabschnitts mit dem vierten Filter-Anschluss vor, wobei an den Auslass der Ultrafiltrationspumpe ein zu einem Behältnis zur Aufnahme von Ultrafiltrat führender Ultrafiltrat-Leitungsabschnitt angeschlossen ist. Das Ultrafiltrat kann somit über einen oder beide Filteranschlüsse abgezogen werden. Dabei können die betreffenden Leitungsabschnitte direkt oder über weitere Leitungsabschnitte an die Filteranschlüsse angeschlossen sein. Zur Unterbrechung der Fluidverbindung können in den Leitungsabschnitten auch Absperrventile oder dergleichen vorgesehen sein.

Der extrakorporale Blutkreislauf der Ultrafiltrationseinheit weist eine erste von dem Patienten abgehende arterielle Blutleitung und eine zweite zu dem Patienten führende venöse Blutleitung auf.

Die arterielle Blutleitung führt zu dem ersten Filter-Anschluss des Filters, so dass Blut in die erste Kammer des Filters strömen kann, während die venöse Blutleitung von dem zweiten Filter-Anschluss abgeht, so dass Blut aus der ersten Kammer strömen kann.

Die Blutleitungen sind vorzugsweise Bestandteil eines zur einmaligen Verwendung bestimmten Schlauchsets (Disposable). Vorzugsweise ist die Ultrafiltrationspumpe eine Rollenpumpe, in die sich die vorzugsweise venöse Blutleitung des Schlauchsets einfach einlegen lässt. Hierzu können die Blutleitungen über geeignete Konnektoren verfügen.

Die Ultrafiltrationseinheit weist vorzugsweise eine Steuereinheit für die Blutpumpe und die Ultrafiltrationspumpe auf, die unterschiedlich ausgebildet sein kann, um einen ordnungsgemäßen Betrieb der Pumpen sicherzustellen. Die Steuereinheit kann für die Pumpen beispielsweise die erforderlichen Drehzahlen vorgegeben, wobei die Steuereinheit auch die Drehzahl der Pumpen überwachen kann. Darüber hinaus kann die Steuereinheit auch eine Alarmfunktion übernehmen.

Bei einer besonders bevorzugten Ausführungsform weist die Steuereinheit elektrische Anschlüsse zur Herstellung einer elektrischen Verbindung zwischen der Steuereinheit der Ultrafiltrationseinheit und einer Steuereinheit der Vorrichtung zur Versorgung der Ultrafiltrationseinheit mit Dialysierflüssigkeit auf, so dass die beiden Steuereinheiten miteinander kommunizieren können. Die Steuereinheiten können für den Datenaustausch über geeignete Schnittstellen verfügen. Die elektrischen Anschlüsse können unterschiedlich ausgebildet sein. Zur Herstellung der elektrischen Verbindung ist vorzugsweise eine Andockstation vorgesehen, so dass sich die Ultrafiltrationseinheit einfach und sicher mit der Versorgungsvorrichtung elektrisch verbinden lässt. Die Andockstation umfasst vorzugsweise sämtliche Anschlüsse, die sowohl für die Fluidverbindung als auch die elektrische Verbindung erforderlich sind.

Darüber hinaus weist die Ultrafiltrationseinheit eine Benutzerschnittstelle auf, die unterschiedlich ausgebildet sein kann. Die Benutzerschnittstelle kann eine Eingabeeinheit und eine Anzeigeeinheit aufweisen. Die Eingabeeinheit kann einen oder mehrerer Bedienelemente, beispielsweise Schalter oder Taster, und die Anzeigeeinheit ein Display aufweisen. Die Bedienung kann aber auch mit einem Touchscreen erfolgen. Im einfachsten Fall weist die Eingabeeinheit nur ein Bedienelement zum Einschalten und Ausschalten der Pumpen auf.

Eine weitere besonders bevorzugte Ausführungsform sieht die Ausbildung der Ultrafiltrationseinheit als eine mit Gurten am Körper des Patienten zu tragende Einheit vor, so dass der Patient die Ultrafiltrationseinheit mit sich führen kann, ohne in seiner Bewegung stark eingeschränkt zu sein. Die Ultrafiltrationseinheit kann beispielsweise wie eine Tasche oder ein Rucksack ausgebildet sein. Es ist aber auch möglich, dass die Ultrafiltrationseinheit als eine Einheit ausgebildet ist, die an einem beweglichen Ständer aufgehängt wird. Hierzu kann die Ultrafiltrationseinheit über geeignete Befestigungselemente, beispielsweise Haken oder Ösen verfügen.

Die Ultrafiltrationseinheit weist vorzugsweise eine die Blutpumpe und die Ultrafiltrationspumpe aufnehmende Trägereinheit auf, die auch die weiteren Komponenten der Ultrafiltrationseinheit, beispielsweise die Steuereinheit aufnehmen kann. Das Ultrafiltrat kann in einem Behältnis, beispielsweise in einem Beutel gesammelt werden, den der Patient mit sich tragen kann. Der Beutel wird von dem Patienten vorzugsweise unabhängig von der Trägereinheit getragen. Hier zu kann auch das Behältnis als eine mit Gurten am Körper des Patienten zu tragende Einheit ausgebildet sein.

Im Folgenden wird ein Ausführungsbeispiel der Erfindung unter Bezugnahme auf die Zeichnungen näher erläutert.

Es zeigen:
- Fig. 1A: die erfindungsgemäße Ultrafiltrationseinheit in vereinfachter schematischer Darstellung,
- Fig. 1B: die Ultrafiltrationseinheit von Fig. 1A in der Seitenansicht,
- Fig. 1C: die Ultrafiltrationseinheit von Fig. 1A in der Draufsicht,
- Fig. 2A: die als Seitentasche ausgebildete Trägereinheit der Ultrafiltrationseinheit,
- Fig. 2B: das als Rucksack ausgebildete Behältnis zur Aufnahme des Ultrafiltrats der Ultrafiltrationseinheit und
- Fig.3: die erfindungsgemäße Ultrafiltrationseinheit und die erfindungsgemäße Vorrichtung zur Versorgung der Ultrafiltrationseinheit mit Dialysierflüssigkeit in stark vereinfachter schematischer Darstellung.

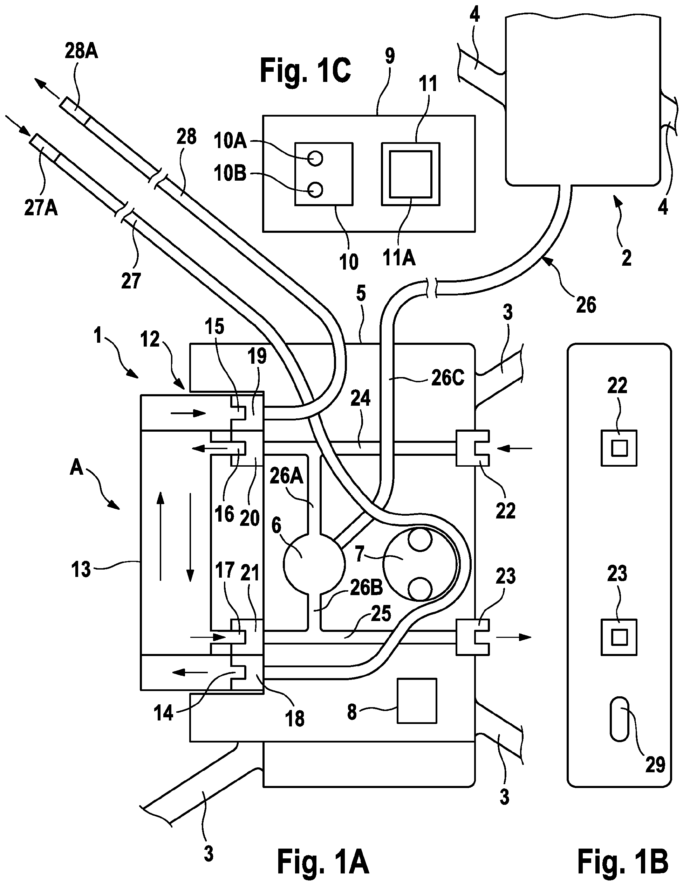

Die Figuren 1A, 1B und 1C zeigen die wesentlichen Komponenten der erfindungsgemäßen Ultrafiltrationseinheit in vereinfachter schematischer Darstellung. Die Ultrafiltrationseinheit umfasst eine Trägereinheit 1 mit den Maschinekomponenten und ein Behältnis 2 zur Aufnahme von Ultrafiltrat.

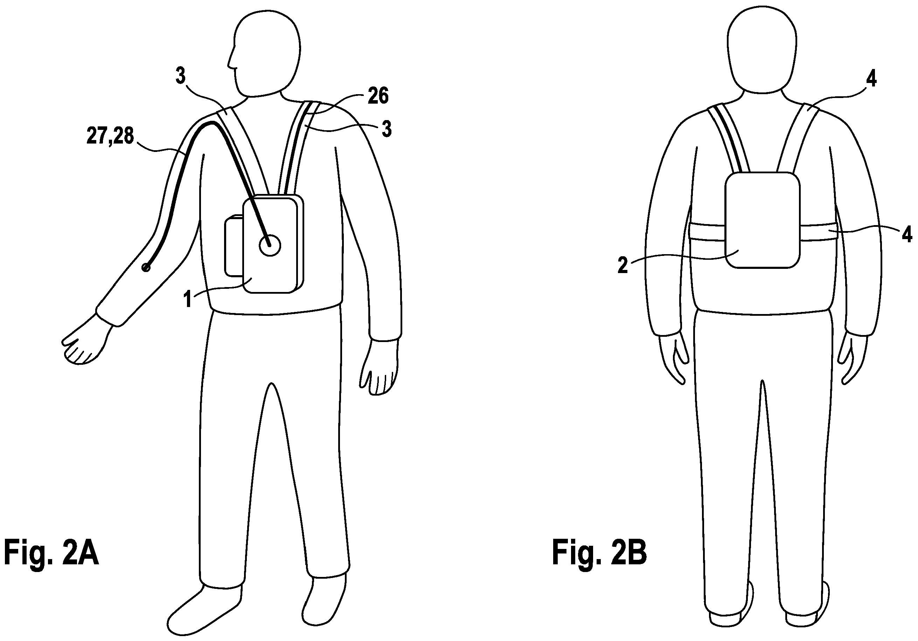

Die Trägereinheit 1 ist in der Art einer Umhänge- oder Seitentasche ausgebildet, die seitliche Tragegurte 3 aufweist, so dass sich der Patient die Trägereinheit 1 umhängen kann. Fig. 2A zeigt den Patienten mit der Trägereinheit 1. Das Behältnis 2 zur Aufnahme des Ultrafiltrats ist in der Art eines Rucksacks ausgebildet, der von dem Patienten an Gurten 4 auf dem Rücken getragen wird. Fig. 2B zeigt den Patienten mit dem Behältnis 2. Das Behältnis 2 kann ein flexibler Kunststoffbeutel sein.

Die Trägereinheit 1 weist einen Gehäusekörper 5 auf, der eine Ultrafitrationspumpe 6 und eine Blutpumpe 7, insbesondere Rollenpumpe, aufnimmt. In dem Gehäusekörper 5 befindet sich auch eine Steuereinheit 8, mit der die Ultrafitrationspumpe 6 und die Blutpumpe 7 angesteuert werden. An der Oberseite des Gehäusekörpers 5 befindet sich eine Benutzerschnittstelle 9, die eine Eingabeeinheit 10 mit Schaltern oder Tastern 10A, 10B und eine Anzeigeeinheit 11 mit einem Display 11A aufweist.

An einer der beiden Längsseiten weist der Gehäusekörper 5 eine Halterung 12 zum Einsetzen eines Filters 13 (Dialysators) auf, der zum einmaligen Gebrauch bestimmt ist. Der Filter 13 kann ein konventioneller Dialysefilter sein, der durch eine semipermeable Membran in eine erste Kammer und eine zweite Kammer unterteilt ist. Die erste Kammer weist einen seitlichen Einlass 14 und Auslass 15 und die zweite Kammer weist einen seitlichen Einlass 16 und Auslass 17 auf. Zum Anschluss des Filters 13 verfügt die Trägereinheit 1 über einen ersten Filter-Anschluss 18 für den Einlass 15 der ersten Kammer des Filters 13 und einen zweiten Filter-Anschluss 19 für den Auslass15 der ersten Kammer des Filters sowie einen dritten Filter-Anschluss 20 für den Einlass 16 der zweiten Kammer des Filters und einen vierten Filter-Anschluss 21 für den Auslass 17 der zweiten Kammer des Filters. Diese Anschlüsse können an einer der beiden Längsseiten des Gehäusekörpers vorgesehen sein. An der gegenüberliegenden Längsseite des Gehäusekörpers befinden sich ein erster Dialysat-Anschluss 22 und zweiter Dialysat-Anschluss 23, so dass die Ultrafiltrationsvorrichtung A an eine in den Figuren 1A bis 1C nicht dargestellte Vorrichtung B zur Versorgung der Ultrafiltrationseinheit mit Dialysierflüssigkeit angeschlossen werden kann. Eine erste Dialysat-Verbindungsleitung 24 verbindet den ersten Dialysat-Anschluss 22 mit dem dritten Filter-Anschluss 20, und eine zweite Dialysat-Verbindungsleitung 25 verbindet den zweiten Dialysat-Anschluss 23 mit dem vierten Filter-Anschluss 17. Von der ersten Dialysat-Verbindungsleitung 24 zweigt ein Leitungsabschnitt 26A einer Ultrafiltrationsleitung 26 ab, und von der zweiten Dialysat-Verbindungsleitung 25 zweigt ein Leitungsabschnitt 26B der Ultrafiltrationsleitung 26 ab. Beide Leitungsabschnitte 26A, 26B der Ultrafiltrationsleitung 26 führen zu dem Einlass der Ultrafiltrationspumpe 6, so dass mit der Ultrafiltrationspumpe 6 Ultrafiltrat aus der zweiten Kammer des Filters 13 abgezogen werden kann. Ein weiterer Leitungsabschnitt 26C der Ultrafiltrationsleitung 26 verbindet den Auslass der Ultrafiltrationspumpe 6 mit dem Einlass des Behältnisses 2 zur Aufnahme des Ultrafiltrats.

Der extrakorporale Blutkreislauf umfasst eine arterielle Blutleitung 27 mit einem arteriellen Patientenanschluss 27A und eine venöse Blutleitung 28 mit einem venösen Patientenanschluss 28A, die Bestandteil eines Schlauchsets sind, das zum einmaligen Gebrauch bestimmt ist. Die arterielle und venöse Blutleitung 27, 28 können direkt mit den ersten und zweiten Filteranschlüssen 18, 19 verbunden sein, d.h. die Filteranschlüsse sind Bestandteil des Disposables oder die Blutleitungen können mit geeigneten Konnektoren versehen sein, die einen Anschluss an fest in dem Gehäusekörper installierte FilterAnschlüsse erlauben. Die arterielle Blutleitung 27 wird in die Rollenpumpe 7 eingelegt, so dass die Rollenpumpe 7 Blut des Patienten durch die erste Kammer (Blutkammer) des Filters 13 fördern und die Ultrafiltrationspumpe 6 Ultrafiltrat aus der zweiten Kammer des Filters abziehen kann.

Die Steuereinheit 8 weist zum Datenaustausch elektrische Anschlüsse 29 auf, die an einer der beiden Längsseiten des Gehäusekörpers 5 vorgesehen sind.

Die Ultrafiltrationseinheit A kann noch weitere Komponenten enthalten, die aber in den Figuren 1A bis 1C nicht dargestellt sind. Beispielsweise kann in der ersten und zweiten Verbindungsleitung 24, 25 jeweils ein Absperrorgan vorgesehen sein, um die Leitungen für den Betrieb der Ultrafiltrationseinheit absperren zu können. Es ist aber auch möglich, dass die Dialysat-Anschlüsse 22, 23 als Anschlüsse ausgebildet sind, die nur dann öffnen, wenn geeignete Konnektoren an die Anschlüsse angeschlossen sind.

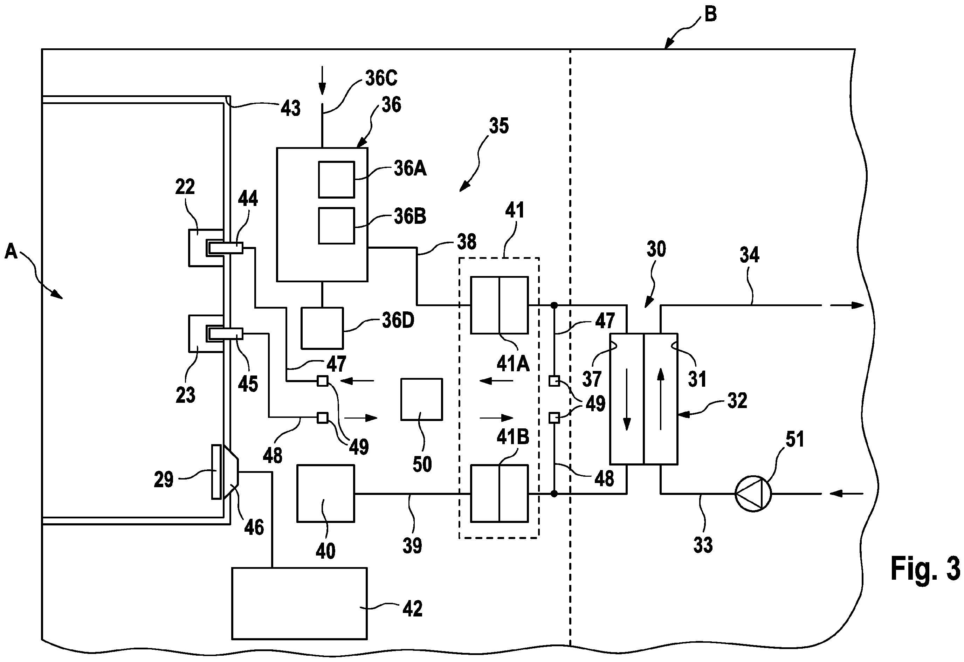

Die Ultrafiltrationseinheit A kann aber auch in Verbindung der erfindungsgemäßen Vorrichtung B zur Versorgung der Ultrafiltrationseinheit mit Dialysierflüssigkeit betrieben werden. Fig. 3 zeigt in stark vereinfachter schematischer Darstellung das medizinische Behandlungssystem, das die Ultrafiltrationseinheit A und die Versorgungsvorrichtung B umfasst. In Fig. 3 sind nur die für die Erfindung wesentlichen Baugruppen der Versorgungvorrichtung B dargestellt.

Die Versorgungsvorrichtung B kann eine Blutbehandlungsvorrichtung sein, die über einen eigenen extrakorporalen Kreislauf verfügt, um einen autarken Betrieb der Versorgungsvorrichtung als Blutbehandlungsvorrichtung zu ermöglichen. Es ist aber auch möglich, dass die Versorgungsvorrichtung nur die für die Versorgung der Ultrafiltrationseinheit erforderlichen Baugruppen verfügt.

Bei dem vorliegenden Ausführungsbeispiel weist die Versorgungsvorrichtung B einen extrakorporalen Blutkreislauf 30 auf, der aber für den Betrieb der Ultrafiltrationseinheit A in Verbindung mit der Versorgungsvorrichtung ungenutzt bleibt. Der extrakorporale Blutkreislauf 30 umfasst eine zu der Blutkammer 31 eines Dialysators 32 führende arterielle Blutleitung 33 und von der Blutkammer abgehende venöse Blutleitung 34. Zum Fördern des Blutes im extrakorporalen Blutkreislauf 30 ist in der arteriellen Blutleitung 33 eine Blutpumpe 51 vorgesehen.

Das Dialysierflüssigkeitssystem 35 der Versorgungsvorrichtung B weist eine Einrichtung 36 zur Bereitstellung von frischer Dialysierflüssigkeit auf, die unterschiedlich ausgebildet sein kann. Bei dem vorliegenden Ausführungsbeispiel weist die Einrichtung 36 zur Bereitstellung von frischer Dialysierflüssigkeit eine Mischeinheit 36A zum Mischen von RO-Wasser und Konzentrat und eine Heizeinheit 36B zum Aufheizen der Dialysierflüssigkeit auf. Das RO-Wasser kann über einen Wasseranschluss 36C zugeführt und das Konzentrat in einem Konzentratbehälter 36D bereitgestellt werden. Die Einrichtung 36 zur Bereitstellung von frischer Dialysierflüssigkeit kann noch über weitere bekannte Komponenten verfügen, beispielsweise eine Einheit zum Entgasen.

Das Dialysierflüssigkeitssystem 35 der autark als Blutbehandlungsvorrichtung betreibbaren Versorgungsvorrichtung B weist eine zu der Dialysierflüssigkeitskammer 37 des Dialysators 32 führende Dialysierflüssikeitszuführleitung 38 und eine von der Dialysierflüssigkeitskammer des Dialysators abgehende Dialysierflüssikeitsabführleitung 39 auf, die zu einen Abfluss oder einen Behälter 40 führt. Darüber hinaus weist das Dialysierflüssigkeitssystem 35 eine Bilanzierungseinheit 41 zum Bilanzieren von frischer und verbrauchter Dialysierflüssigkeit auf, die über Bilanzkammern 41A, 41B verfügen kann.

Für die Steuerung sämtlicher Maschinenkomponenten ist eine zentrale Steuereinheit 42 vorgesehen.

Zur Versorgung der Ultrafiltrationseinheit mit Dialysierflüssigkeit werden nur die Einrichtung 36 zur Bereitstellung von frischer Dialysierflüssigkeit mit der Misch- und Heizeinheit 36A, 36B sowie dem Konzentratbehälter 36C und die Bilanzierungseinheit 41 und die Steuereinheit 42 benötigt.

Zum Anschluss der als austauschbares Modul ausgebildeten Ultrafiltrationseinheit A weist die Versorgungsvorrichtung B eine Andockstation 43 auf, mit der sich sämtliche Anschlüsse herstellen lassen. Die Andockstation 43 weist einen ersten und zweiten Konnektor 44, 45 für den ersten bzw. zweiten Dialysat-Anschluss 22, 23 der Ultrafiltrationseinheit A und einen elektrisches Anschlussstück 46 für die elektrischen Anschlüsse 29 der Ultrafiltrationseinheit auf. Der erste Konnektor 44 ist mit einer Zuführleitung 47 für frische und der zweite Anschluss 45 ist mit einer Abführleitung 48 für verbrauchte Dialysierflüssigkeit verbunden. Die Zuführ- und Abführleitung 47, 48 sind an in Fig. 3 nur andeutungsweise dargestellten Anschlussstellen 49 des Dialysierflüssigkeitssystems 35 angeschlossen, an denen frische Dialysierflüssigkeit dem Dialysierflüssigkeitssystem entnommen bzw. verbrauchte Dialysierflüssigkeit dem Dialysierflüssigkeitssystem zugeführt werden kann. In dem Dialysierflüssigkeitssystem wird die frische Dialysierflüssigkeit mit der Einrichtung 36 zur Bereitstellung frischer Dialysierflüssigkeit bereitgestellt und in den Abfluss oder Behälter 40 abgeführt, wobei die frische und verbrauchte Dialysierflüssigkeit mit der Bilzanzierungseinheit 41 bilanziert werden. Für den Betrieb mit der Ultrafiltrationseinheit ist eine in Fig. 3 nur andeutungsweise dargestellte Ventileinrichtung 50 vorgesehen, die derart ausgebildet ist, dass der extrakorporale Blutkreislauf 30 abgetrennt wird.

Wenn die Ultrafiltrationseinheit A an die Versorgungsvorrichtung B angeschlossen ist (Fig. 3) strömt frische Dialysierflüssigkeit durch die zweite Kammer (Dialysierflüssigkeitskammer) des Filters 13 der Ultrafiltrationseinheit A, während Ultrafiltrat aus der zweiten Kammer über die Ultrafiltrationsleitung 26 abgezogen wird. Folglich ist die Ultrafiltrationseinheit A universell einsetzbar und erlaubt nicht nur eine Ultrafiltration zwischen den Dialysebehandlungen, sondern in Verbindung mit der Versorgungseinrichtung B auch eine Dialysebehandlung.

## Patentansprüche

1. Medizinisches Behandlungssystem mit einer portablen Ultrafiltrationseinheit (A) und einer stationären Vorrichtung (B) zur Versorgung der Ultrafiltrationseinheit mit Dialysierflüssigkeit, wobei
die portable Ultrafiltrationseinheit eine Blutpumpe (7) zum Fördern von Blut und eine Ultrafiltrationspumpe (6) zum Fördern von Ultrafiltrat aufweist und als eine Einheit (A) zum Anschluss an die stationäre Vorrichtung (B) zur Versorgung der Ultrafiltrationseinheit mit Dialysierflüssigkeit derart ausgebildet ist, dass eine Flüssigkeitsverbindung für die Zufuhr von frischer und Abfuhr von verbrauchter Dialysierflüssigkeit herstellbar ist,
**dadurch gekennzeichnet, dass**
die stationäre Vorrichtung (B) zur Versorgung der Ultrafiltrationseinheit mit Dialysierflüssigkeit eine Einrichtung (36) zur Bereitstellung von frischer Dialysierflüssigkeit, eine Einrichtung (40) zum Abführen von verbrauchter Dialysierflüssigkeit und eine Bilanzierungseinheit (41) zum Bilanzieren von frischer und verbrauchter Dialysierflüssigkeit aufweist, wobei die Vorrichtung (B) zur Versorgung der Ultrafiltrationseinheit (A) derart ausgebildet ist, dass eine Flüssigkeitsverbindung für die Zufuhr von frischer Dialysierflüssigkeit aus der Einrichtung (36) zur Bereitstellung von frischer Dialysierflüssigkeit zu der Ultrafiltrationseinheit (A) und der Abfuhr von verbrauchter Dialysierflüssigkeit von der Ultrafiltrationseinheit (A) in die Einrichtung (40) zum Abführen von verbrauchter Dialysierflüssigkeit herstellbar ist, so dass mit der Ultrafiltrationseinheit (A) eine Dialysebehandlung durchführbar ist.

2. Medizinisches Behandlungssystem nach Anspruch 1, **dadurch gekennzeichnet, dass** die Ultrafiltrationseinheit (A) einen ersten Dialysat-Anschluss (22) für eine Zuführleitung (47) für frisches Dialysat und einen zweiten Dialysat-Anschluss (23) für eine Abführleitung (48) für verbrauchtes Dialysat aufweist.

3. Medizinisches Behandlungssystem nach Anspruch 1 oder 2, **dadurch gekennzeichnet, dass** die Ultrafiltrationseinheit (A) einen ersten Filter-Anschluss (18) für einen Einlass einer ersten Kammer eines Filters (13), der durch eine semipermeable Membran in die erste und eine zweite Kammer unterteilt ist, und einen zweiten Filter-Anschluss (19) für einen Auslass (15) der ersten Kammer des Filters aufweist, und einen dritten Filter-Anschluss (20) für einen Einlass (16) der zweiten Kammer des Filters und einen vierten Filter-Anschluss (21) für einen Auslass (17) der zweiten Kammer des Filters aufweist.

4. Medizinisches Behandlungssystem nach Anspruch 3, **dadurch gekennzeichnet, dass** eine erste Dialysat-Verbindungsleitung (24) vorgesehen ist, die den ersten Dialysat-Anschluss (22) mit dem dritten Filter-Anschluss (20) verbindet, und eine zweite Dialysat-Verbindungsleitung (25) vorgesehen ist, die den zweiten Dialysat-Anschluss (23) mit dem vierten Filter-Anschluss (21) verbindet.

5. Medizinisches Behandlungssystem nach Anspruch 3 oder 4, **dadurch gekennzeichnet, dass** ein zu dem Einlass der Ultrafiltrationspumpe (6) führender Ultrafiltrat-Leitungsabschnitt (26A) mit dem dritten Filter-Anschluss (20) und/oder ein zu dem Einlass der Ultrafiltrationspumpe führender Ultrafiltrat-Leitungsabschnitt (26B) mit dem vierten Filter-Anschluss (21) in Fluidverbindung steht, wobei an den Auslass der Ultrafiltrationspumpe (6) ein zu einem Behältnis (2) zur Aufnahme von Ultrafiltrat führender Ultrafiltrat-Leitungsabschnitt (26C) angeschlossen ist.

6. Medizinisches Behandlungssystem nach einem der Ansprüche 3 bis 5, **dadurch gekennzeichnet, dass** die Ultrafiltrationseinheit (A) eine zu dem ersten Filter-Anschluss (19) führende erste Blutleitung (27) zum Zuführen von Blut in die erste Kammer des Filters (13) und eine von dem zweiten Filter-Anschluss (18) abgehende Blutleitung zum Abführen von Blut aus der ersten Kammer des Filters (13) aufweist.

7. Medizinisches Behandlungssystem nach einem der Ansprüche 1 bis 6, **dadurch gekennzeichnet, dass** die Ultrafiltrationseinheit (A) eine Steuereinheit (8) für die Blutpumpe (7) und die Ultrafiltrationspumpe (6) aufweist.

8. Medizinisches Behandlungssystem nach einem der Ansprüche 1 bis 7, **dadurch gekennzeichnet, dass** die Steuereinheit (8) elektrische Anschlüsse (29) zur Herstellung einer elektrischen Verbindung zwischen der Steuereinheit (8) der Ultrafiltrationseinheit (A) und einer Steuereinheit (42) der Vorrichtung (B) zur Versorgung der Ultrafiltrationseinheit mit Dialysierflüssigkeit aufweist.

9. Medizinisches Behandlungssystem nach einem der Ansprüche 1 bis 8, **dadurch gekennzeichnet, dass** die Ultrafiltrationseinheit (A) eine Benutzerschnittstelle (9) aufweist.

10. Medizinisches Behandlungssystem nach einem der Ansprüche 1 bis 9, **dadurch gekennzeichnet, dass** die Ultrafiltrationseinheit (A) als eine mit Gurten am Körper des Patienten zu tragende Einheit ausgebildet ist.

11. Medizinisches Behandlungssystem nach einem der Ansprüche 1 bis 10, **dadurch gekennzeichnet, dass** die Ultrafiltrationseinheit (A) eine die Blutpumpe (7) und die Ultrafiltrationspumpe (6) aufnehmende Trägereinheit (1) aufweist.

12. Medizinisches Behandlungssystem nach einem der Ansprüche 1 bis 11, **dadurch gekennzeichnet, dass** die Vorrichtung (B) zur Versorgung der Ultrafiltrationseinheit mit Dialysierflüssigkeit eine Andockstation (43) zur Herstellung einer Flüssigkeitsverbindung für die Zufuhr von frischer Dialysierflüssigkeit zu der Ultrafiltrationseinheit (A) und Abfuhr von verbrauchter Dialysierflüssigkeit von der Ultrafiltrationseinheit aufweist.

13. Medizinisches Behandlungssystem nach einem der Ansprüche 1 bis 12, **dadurch gekennzeichnet, dass** die Vorrichtung (B) zur Versorgung der Ultrafiltrationseinheit mit Dialysierflüssigkeit als eine Vorrichtung zur extrakorporalen Blutbehandlung ausgebildet ist, die einen extrakorporalen Blutkreislauf (30) und ein Dialysierflüssigkeitssystem (35) aufweist.

## Claims

1. Medical treatment system comprising a portable ultrafiltration unit (A) and a stationary device (B) for supplying dialysate to the ultrafiltration unit,
the portable ultrafiltration unit comprising a blood pump (7) for conveying blood and an ultrafiltration pump (6) for conveying ultrafiltrate and being in the form of a unit (A) to be connected to the stationary device (B) for supplying dialysate to the ultrafiltration unit, such that a fluid connection can be established for feeding in fresh dialysate and removing used dialysate,
**characterised in that**
the stationary device (B) for supplying dialysate to the ultrafiltration unit comprises an apparatus (36) for providing fresh dialysate, an apparatus (40) for removing used dialysate, and a balancing unit (41) for balancing fresh and used dialysate, wherein the device (B) for supplying the ultrafiltration unit (A) is designed such that a fluid connection can be established for feeding fresh dialysate from the apparatus (36) for providing fresh dialysate to the ultrafiltration unit (A) and removing used dialysate from the ultrafiltration unit (A) to the apparatus (40) for removing used dialysate so that a dialysis treatment can be carried out using the ultrafiltration unit.

2. Medical treatment system according to claim 1, **characterised in that** the ultrafiltration unit (A) comprises a first dialysate connection (22) for a feed line (47) for fresh dialysate and a second dialysate connection (23) for a removal line (48) for used dialysate.

3. Medical treatment system according to either claim 1 or claim 2, **characterised in that** the ultrafiltration unit (A) comprises a first filter connection (18) for an inlet of a first chamber of a filter (13), which is divided into the first chamber and a second chamber by a semipermeable membrane, and a second filter connection (19) for an outlet (15) of the first chamber of the filter, and comprises a third filter connection (20) for an inlet (16) of the second chamber of the filter and a fourth filter connection (21) for an outlet (17) of the second chamber of the filter.

4. Medical treatment system according to claim 3, **characterised in that** a first dialysate connection line (24) is provided which connects the first dialysate connection (22) to the third filter connection (20), and a second dialysate connection line (25) is provided which connects the second dialysate connection (23) to the fourth filter connection (21).

5. Medical treatment system according to either claim 3 or claim 4, **characterised in that** an ultrafiltrate line portion (26A) which leads to the inlet of the ultrafiltration pump (6) is in fluid communication with the third filter connection (20) and/or an ultrafiltrate line portion (26B) which leads to the inlet of the ultrafiltration pump is in fluid communication with the fourth filter connection (21), an ultrafiltrate line portion (26C) which leads to a container (2) for holding ultrafiltrate being connected to the outlet of the ultrafiltration pump (6).

6. Medical treatment system according to any of claims 3 to 5, **characterised in that** the ultrafiltration unit (A) comprises a first blood line (27) leading to the first filter connection (19) for feeding blood into the first chamber of the filter (13) and a blood line leading away from the second filter connection (18) for removing blood from the first chamber of the filter (13).

7. Medical treatment system according to any of claims 1 to 6, **characterised in that** the ultrafiltration unit (A) comprises a control unit (8) for the blood pump (7) and the ultrafiltration pump (6).

8. Medical treatment system according to any of claims 1 to 7, **characterised in that** the control unit (8) comprises electrical connections (29) for producing an electrical connection between the control unit (8) of the ultrafiltration unit (A) and a control unit (42) of the device (B) for supplying dialysate to the ultrafiltration unit.

9. Medical treatment system according to any of claims 1 to 8, **characterised in that** the ultrafiltration unit (A) comprises a user interface (9).

10. Portable ultrafiltration unit according to any of claims 1 to 9, **characterised in that** the ultrafiltration unit is in the form of a unit to be worn on the patient's body by means of straps.

11. Medical treatment system according to any of claims 1 to 10, **characterised in that** the ultrafiltration unit (A) comprises a carrier unit (1) which houses the blood pump (7) and the ultrafiltration pump (6).

12. Medical treatment system according to any of claims 1 to 11, **characterised in that** the device (B) for supplying dialysate to the ultrafiltration unit comprises a docking station (43) for establishing a fluid connection for feeding fresh dialysate to the ultrafiltration unit (A) and removing used dialysate from the ultrafiltration unit.

13. Medical treatment system according to any of claims 1 to 12, **characterised in that** the device (B) for supplying dialysate to the ultrafiltration unit is in the form of a device for extracorporeal blood treatment which comprises an extracorporeal blood circuit (30) and a dialysate system (35).

## Revendications

1. Système de traitement médical avec une unité d'ultrafiltration portable (A) et un dispositif fixe (B) pour alimenter l'unité d'ultrafiltration avec du liquide de dialyse, dans lequel
l'unité d'ultrafiltration portable présente une pompe à sang (7) pour transporter du sang et une pompe d'ultrafiltration (6) pour transporter de l'ultrafiltrat et est conçue comme une unité (A) pour le branchement au dispositif fixe (B) pour alimenter l'unité d'ultrafiltration avec du liquide de dialyse de sorte qu'une liaison par liquide pour l'apport de liquide de dialyse frais et l'évacuation de liquide de dialysé usé peut être établie,
**caractérisé en ce que**
le dispositif fixe (B) pour alimenter l'unité d'ultrafiltration avec du liquide de dialyse présente un dispositif (36) pour fournir du liquide de dialyse frais, un dispositif (40) pour évacuer le liquide de dialysé usé et une unité d'établissement de bilan (41) pour établir le bilan du liquide de dialyse frais et usé, dans lequel le dispositif (B) pour alimenter l'unité d'ultrafiltration (A) est conçu de sorte qu'une liaison par liquide pour l'apport de liquide de dialyse frais depuis le dispositif (36) pour fournir du liquide de dialyse frais à l'unité d'ultrafiltration (A) et l'évacuation du liquide de dialysé usé de l'unité d'ultrafiltration (A) dans le dispositif (40) pour évacuer le liquide de dialysé usé peut être établie, de sorte qu'avec l'unité d'ultrafiltration (A) un traitement de dialyse peut être réalisé.

2. Système de traitement médical selon la revendication 1, **caractérisé en ce que** l'unité d'ultrafiltration (A) présente un premier branchement de dialysat (22) pour une ligne d'apport (47) pour le dialysat frais et un second branchement de dialysat (23) pour une ligne d'évacuation (48) pour le dialysat usé.

3. Système de traitement médical selon la revendication 1 ou 2, **caractérisé en ce que** l'unité d'ultrafiltration (A) présente un premier branchement de filtre (18) pour une entrée d'une première chambre d'un filtre (13) qui est subdivisé par une membrane semi-perméable en la première et une seconde chambre, et un second branchement de filtre (19) pour une sortie (15) de la première chambre du filtre, et un troisième branchement de filtre (20) pour une entrée (16) de la seconde chambre du filtre et un quatrième branchement de filtre (21) pour une sortie (17) de la seconde chambre du filtre.

4. Système de traitement médical selon la revendication 3, **caractérisé en ce qu'**une première ligne de liaison de dialysat (24) qui relie le premier branchement de dialysat (22) au troisième branchement de filtre (20) est prévue, et une seconde ligne de liaison de dialysat (25) qui relie le second branchement de dialysat (23) au quatrième branchement de filtre (21) est prévue.

5. Système de traitement médical selon la revendication 3 ou 4, **caractérisé en ce qu'**une section de ligne d'ultrafiltrat (26A) conduisant à l'entrée de la pompe d'ultrafiltration (6) est en liaison par fluide avec le troisième branchement de filtre (20) et/ou une section de ligne d'ultrafiltrat (26B) conduisant à l'entrée de la pompe d'ultrafiltration est en liaison par fluide avec le quatrième branchement de filtre (21), dans lequel une section de ligne d'ultrafiltrat (26C) conduisant à un récipient (2) pour recevoir l'ultrafiltrat est branchée à la sortie de la pompe d'ultrafiltration (6).

6. Système de traitement médical selon l'une des revendications 3 à 5, **caractérisé en ce que** l'unité d'ultrafiltration (A) présente une première ligne de sang (27) conduisant au premier branchement de filtre (19) pour apporter du sang à la première chambre du filtre (13) et une conduite de sang partant du second branchement de filtre (18) pour évacuer le sang de la première chambre du filtre (13).

7. Système de traitement médical selon l'une des revendications 1 à 6, **caractérisé en ce que** l'unité d'ultrafiltration (A) présente une unité de commande (8) pour la pompe à sang (7) et la pompe d'ultrafiltration (6).

8. Système de traitement médical selon l'une des revendications 1 à 7, **caractérisé en ce que** l'unité de commande (8) présente des branchements électriques (29) pour établir une liaison électrique entre l'unité de commande (8) de l'unité d'ultrafiltration (A) et une unité de commande (42) du dispositif (B) pour l'alimentation de l'unité d'ultrafiltration avec du liquide de dialyse.

9. Système de traitement médical selon l'une des revendications 1 à 8, **caractérisé en ce que** l'unité d'ultrafiltration (A) présente une interface utilisateur (9).

10. Système de traitement médical selon l'une des revendications 1 à 9, **caractérisé en ce que** l'unité d'ultrafiltration (A) est conçue comme une unité à porter avec des sangles sur le corps du patient.

11. Système de traitement médical selon l'une des revendications 1 à 10, **caractérisé en ce que** l'unité d'ultrafiltration (A) présente une unité de support (1) qui reçoit la pompe à sang (7) et la pompe d'ultrafiltration (6).

12. Système de traitement médical selon l'une des revendications 1 à 11, **caractérisé en ce que** le dispositif (B) pour alimenter l'unité d'ultrafiltration avec du liquide de dialyse présente une station d'accueil (43) pour réaliser une liaison par liquide pour l'apport de liquide de dialyse frais à l'unité d'ultrafiltration (A) et l'évacuation du liquide de dialyse usé de l'unité d'ultrafiltration.

13. Système de traitement médical selon l'une des revendications 1 à 12, **caractérisé en ce que** le dispositif (B) pour alimenter l'unité d'ultrafiltration avec du liquide de dialyse est conçu comme un dispositif de traitement du sang extracorporel, qui présente un circuit de sang extracorporel (30) et un système de liquide de dialyse (35).
